(19) Europäisches Patentamt
European Patent Office
Office européen des brevets

(11) Numéro de publication: 0 233 801
B1

(12) **FASCICULE DE BREVET EUROPEEN**

(45) Date de publication du fascicule du brevet: 23.05.90

(21) Numéro de dépôt: 87400079.7

(22) Date de dépôt: 15.01.87

(51) Int. Cl.[5]: C 07 D 471/04, A 61 K 31/435, A 61 K 31/495, A 61 K 31/50 // (C07D471/04, 221:00, 221:00)

(54) **Nouveaux amides substitués, leur préparation et les compositions pharmaceutiques qui les contiennent.**

(30) Priorité: 16.01.86 FR 8600554

(43) Date de publication de la demande: 26.08.87 Bulletin 87/35

(45) Mention de la délivrance du brevet: 23.05.90 Bulletin 90/21

(84) Etats contractants désignés: AT BE CH DE ES FR GB GR IT LI LU NL SE

(56) Documents cités:
EP-A-0 208 621
FR-A-1 006 725

(73) Titulaire: RHONE-POULENC SANTE
20, avenue Raymond Aron
F-92160 Antony (FR)

(72) Inventeur: Cotrel, Claude
17 A. Avenue du Docteur Arnold Netter
F-75012 Paris (FR)
Inventeur: Guyon, Claude
17 bis, avenue Henri Martin
F-94100 Saint-Maur-des-Fosses (FR)
Inventeur: Roussel, Gérard
20 ter, rue des Carrières
F-91450 Soisy-sur-Seine (FR)
Inventeur: Taurand, Gérard
2/124 Allée Jean de la Bruyère
F-94000 Créteil (FR)

(74) Mandataire: Savina, Jacques et al
RHONE-POULENC SANTE, Service Brevets, 20 Avenue Raymond Aron
F-92165 Antony Cédex (FR)

EP 0 233 801 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

Dans la demande de brevet PCT 84 00489, one été décrits des dérivés de benzamides de formule générale:

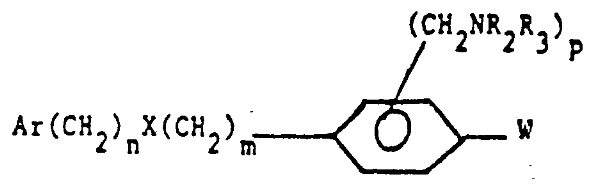

utiles comme antiarythmiques.

Dans la demande de brevet néerlandais 73 05482 et le brevet américain 3 993 656, sont décrits des dérivés de naphtyridine-1,8 de structure:

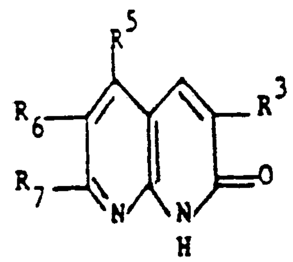

utiles comme brochodilatateurs et vasodilatateurs périphériques ou comme agents hypotenseurs.

La présente invention concerne de nouveaux amides substitués de formulé générale:

$$R—CONH—Het \qquad (I)$$

leur préparation et les médicaments qui les contiennent.

Selon a présente invention, dans la formule générale (I),

soit le symbole R représente un radical phényle substitué par un radical acyloxy, alcoylthio ou alcoyloxycarbonylamino, par un atome de chlore en position -2 ou -4 ou par deux radicaux alcoyloxy, ou représente un radical hétérocyclyle choisi parmi pyridyle-2, pyridyle-4, pyrazinyle, dihydro-5,6 dithiinnyle-2, dihydro-5,6 oxathinnyle-2, dithiolyle-1,3, thiazolyle ou, thiényle ou furyle substitués par un atome halogène ou par un radical alcoylthio, ou R représente un radical alcényle contenant 2 à 4 atomes de carbone et dont la double liaison se trouve en α par rapport au groupement carbonyle de la fonction amide, et le symbole Het représente un radical naphtyridine-1,8 yle-2 substitué en position -7 par une atome d'halogène ou par un radical alcoyloxy, phénoxy, chloro-3 phénoxy, dichlorophénoxy, hydroxy ou cyano,

soit le symbole R représente un radical méthoxyphényle et le symbole Het représente un radical naphtyridine-1,8 yle-2 substitué en position -7 par un radical chloro-3 phénoxy, dichlorophénoxy, hydroxy ou cyano,

soit le symbole R représente un radical méthoxy pyridazinyle-3 et le symbole Het représente un radical naphtyridine-1,8 yle-2 substitué en position -7 par un radical phénoxy,

étant entendu que les radicaux alcoyle et acyle cités ci-dessus contiennent 1 à 4 atomes de carbone en chaîne droite ou ramifiée.

Lorsque le symbole Het contient un substituant halogène, celui-ci est choisi parmi le fluor, le chlore et le brome.

Lorsque le symbole R contient un substituant halogène, ce dernier peut également être choisi parmi le fluor, le chlore et le brome.

Selon l'invention, les produits de formule générale (I) peuvent être obtenus par action d'un acide de formule générale:

$$R—COOH \qquad (II)$$

dans laquelle R est défini comme précédemment, ou d'un dérivé réactif de cet cide, sur une amine de formule générale:

$$H_2N—Het \qquad (III)$$

dans laquelle Het est défini comme précédemment.

Lorsque l'on met en oeuvre l'acide de formule générale (II), on opère en présence d'un agent de condensation peptidique tel qu'un carbodiimide (par exemple le dicyclohexylcarbodiimide) ou le N,N'-carbonyldiimidazole ou l'éthoxy-2-éthoxycarbonyl-1 dihydro-1,2 quinoléine, dans un solvant organique tel qu'un éther (par exemple tétrahydrofuranne, dioxanne, glyme, diglyme), un amide (par exemple diméthylformamide), un nitrile (par exemple acétonitrile) ou un solvant chloré (pare exemple chlorure de méthylène, dichloroéthane ou chloroforme) à une température comprise entre 0°C et la température de reflux du mélange réactionnel. De préférence on opère à 20°C.

Lorsque l'on met en oeuvre un dérivé réactif de l'acide de formule générale (II) il est possible de faire réagir l'anhydride, un anhydride mixte, un halogénure d'acide ou un ester [qui peut être choisi parmi les esters activés ou non de l'acide de formule général (III)]. On opére alors soit en milieu organique, éventuellement en présence d'un accepteur d'acide tel qu'une base organique azotée (par exemple un trialcoylamine, une pyridine, le diaza-1,8 bicyclo [5.4.0] undecène-7 ou le diaza-1,5- bicyclo [4.3.0] nonène-5), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'une carbonate ou bicarbonate d'un métal alcalin ou alcalino terreux à une température comprise entre 0 et 40°C. Il est également possible d'opérer sans solvant à la température de fusion de mélange réactionnel.

Selon l'invention, les amides de formule générale (I) dans laquelle Het est un radical naphtyridine-1,8 yle-2 substitué en position -7 par un radical hydroxy, peuvent également être préparés à partir de l'amide correspondant de formule généale (I) dans laquelle Het est un radical naphtyridine-1,8 yle-2 substitué en position -7 par un atome d'halogène (de préférence un atome de chlore), par traitement en milieu acide, suivi de l'hydrolyse du produit obtenu.

On opère avantageusement par traitement par l'acide acétique éventuellement en présence d'anhydride acétique ou par l'acide formique à une température comprise entre 50°C et la température de reflux de mélange réactionnel.

Les acides de formule générale (II) et leurs dérivés réactifs, peuvent être préparés par application des méthodes décrites ci-dessous (ou par analogie avec ces méthodes) ou selon les méthodes décrites ci-après dans les exemples:

lorsque R est acyloxyphényle, selon Von H. Schönenberger et coll., Arz. Forsch, *14,* 324 (1964);

lorsque R est dihydro-5,6 dithiinnyle-2, selon K. G. Gundermann et coll., *95,* 2076 (1962);

lorsque R est dithiolyle-1,3 selon C. H. Pittman, J.C.S. Chem. Comm., 960 (1975);

lorsque R est thiazolyle H. Erlenmeyer det coll., Helv. CHim. Acta, *20,* 204 (1937) et *25,* 1073 (1942);

lorsque R est thiényle substitué, selon H. D. Hartough, The Chemistry of Heterocyclic compounds, thiophen and its derivatives, Interscience Publishers Inc., New-York, p. 363 (1952);

lorsque R est furyl substitué, selon les méthodes décrites dans Advances in Het. Chem., *7,* 377 (1966) et *30,* 167 (1982).

Les amines de formule générale (III) selon la présente invention dans laquelle Het porte un substituant alcoyloxy ou phénoxy éventuellement substitué, peuvent être preparées à partir de l'amine correspondant dans laquelle le symbole Het porte un substituant halogène (de préférence un atome de chlore) par action d'un dérivé hydroxylé de formule générale:

R'OH (IV)

(dans laquelle R' représente un radical alcoyle, phényle, chloro-3 phényle ou dichlorophényle) en milieu basique, ou par action de l'alcoolate correspondant.

La réaction s'effectue généralement en présence d'une base forte (soude, potasse, hydroxyde d'ammonium quaternaire, éthylate de sodium par exemple), à une température comprise entre 50 et 150°C, ou bien en présence de l'alcoolate correspondant (par exemple l'acoolate de sodium), dans un solvant tel qu'un amide (diméthylformamide par exemple) ou un éther (tétrahydrofuranne, diméthoxyéthane par exemple), ou sans solvant, en présence d'un excés de dérivé hydroxylé à une température comprise entre 70°C et la température de réflux du mélange réactionnel.

Lorsque l'on met en oeuvre l'alcoolate, celui-ci est obtenu préalablement, par action du sodium sur l'alcool de formule générale (IV) à une température comprise entre 20 et 80°C, ou par action de l'hydrure de sodium à un température comprise ente 0 et 20°C dans un solvant tel que le diméthylformamide, le diméthoxyéthane, ou le tétrahydrofuranne. Il n'est par nécessaire d'isoler l'alcoolate obtenu pour le mettre en oeuvre dans la réaction suivante.

Les amines de formule générale (III) peuvent également être prépares par application des méthodes décrites ci-après dans les exemples ou selon la méthode décrite par S. CARBONI, Gazz. Chim. Italiana, *96,* 1456 (1966).

Les nouveaux amides selon la présente invention peuvent être purifiés le cas échéant, par des méthodes physiques telles que la cristallisation ou la chromatographie.

Les produits de formule générale (I) présentent des propriétés pharmacologiques particulièrement intéressantes. Ils manifestent une activité comme anxiolytiques, hypnotiques, et myorelaxants de bon niveau.

Ils présentent notamment une bonne affinité in vitro pour les sites récepteurs à benzodiazépine à des concentrations comprises entre 5 et des valeurs voisines de 1000 nM selon la technique décrite par J. C. Blanchard et L. Julou, J. of Neurochemistry, *40,* 601 (1983) inspirée des travaux de Squires et Braestrup, Nature, *266,* 732 (1977);

Par ailleurs les produits selon l'invention présentent une faible toxicité: leur $DL_{50}$ par voie orale chez le souris est égale ou supérieure à 900 mg/kg.

D'un intérêt particulier sont les produits de formule générale (I) dans laquelle:

soit le symbole R représente un radical phényle substitué par un radical acyloxy, alcoylthio ou alcoyloxycarbonylamino, par un atome de chlore en position -2 ou -4 ou par 2 radicaux alcoyloxy, ou représente un radical hétérocyclyle choisi parmi pyridyle-2, pyridyle-4, pyrazinyle, dihydro-5,6 dithiinnyle-2, dihydro-5,6 oxathiinnyle-2, dithiolyle-1,3, thiazolyle, thiényle-2 ou furyle substitués par un atome halogène en position -5, ou alcoylthio-5 thiényle-2, ou R représente un radical alcényle contenant 2 à 4 atomes de carbone et dont la double liaison se trouve en α par rapport au groupement carbonyle de la fonction amide, et le symbole Het représente un radical naphtyridine-1,8 yle-2 substitué en position-7 par une atome d'halogène ou par un radical alcoyloxy ou phénoxy,

soit le symbole R représente un radical méthoxyphényle et le symbole Het représente un radical naphtyridine-1,8 yle-2 substitué en position -7 par un radical chloro-3 phénoxy, dichloro-3,4 phénoxy, hydroxy ou cyano,

soit le symbole R représente un radical méthoxy-6 pyridazinyle-3 et le symbole Het représente un radical naphtyridine-1,8 yle-2 substitué en position -7 par un radical phénoxy,

étant entendu que les radicaux alcoyle et acyle cités ci-dessus contiennent 1 à 3 atomes de carbone en chaîne droite ou ramifiée.

Et parmi ces produits plus spécialement intéressants sont les amides substitués de formule générale (I) pour lesquels:

soit le symbole R représente un radical phényle substitué par un radical acyloxy ou représente un radical hétérocyclyle choisi parmi dihydro-5,6 dithiinnyle-2, dithiolyle-1,3 ou thiazolyle-4 et le symbole Het représente un radical naphtyridine-1,8 yle-2 substitué en position -7 par une atome d'halogène ou par un radical methoxy,

soit le symbole R représente un radical méthoxy-4 phényle et le symbole Het représent un radical naphtyridine-1,8 yle-2 substitué en position -7 par un radical chloro-3 phénoxy ou cyano.

Et notamment les produits suivants:

N-(choro-7 naphtyridine-1,8 yl-2) acétoxy-4 benzamide
N-(choro-7 naphtyridine-1,8 yl-2) acétoxy-3 benzamide
N-(méthoxy-7 naphtyridine-1,8 yl-2) acétoxy-3 benzamide
N-(chloro-7 naphtyridine-1,8 yl-2) dithiole-1,3 benzamide
N-(cyano-7 naphtyridine-1,8 yl-2) méthoxy-4 benzamide

Les exemples suivants illustrate la présente invention.

Exemple 1

A une solution de 14,1 g d'acide chloro-4 benzoïque dans 90 cm3 de tétrahydrofuranne anhydre, on ajoute 14,7 g de N,N'-carbonyldiimidazole. Le mélange est agité pendant 1 heure à une température voisine de 20°C, jusqu'à la find du dégagement gazeux. On ajoute ensuite 10,8 g d'amino-2 chloro-7 naphtyridine-1,8 et on chauffe à reflux pendant 3 heures.

Le produit insoluble est séparé par filtration, lavé avec 3 fois 50 cm3 de tétrahydrofuranne et séché à 50°C sous pression réduite (0,07 kPa). Après recristallisation du produit ainsi obtenu dans 100 cm3 de propanol-2, on obtient 9,3 g de N- (chloro-7 naphtyridine-1,8 yl-2) chloro-4 benzamide fondant à 268°C.

L'amino-2 chloro-7 naphtyridine-1,8 peut être préparée selon la méthode décrite par S. CARBONI, Gazz. Chim. Italiana, *96,* 1456 (1966).

Exemple 2

A une solution de 12,5 g d'acide chloro-4 benzoïque dans 250 cm3 de tétrahydrofuranne anhydre, on ajoute 12,9 g de N,N'-carbonyldiimidazole. Le mélange est agité pendant 2 heures à une température voisine de 20°C, jusqu'à la fin du dégagement gazeux. On ajoute ensuite 8,9 g d'amino-2 chloro-7 naphtyridine-1,8 et on chauffe à reflux pendant 22 heures. Le mélange réactionnel est versé sur 2000 cm3 d'eau distillée, le précipité formé est séparé par filtration, lavé à l'eau et séché à l'air. Par recristallisation du produit ainsi obtenu, dans 180 cm3 d'éthanol, on obtient 6,9 g de N-(chloro-7 naphtyridine-1,8 yl-2) chloro-4 benzamide fondant à 180°C.

Exemple 3

On ajoute goutte à goutte 23,5 g de chlorure d'acétoxy-4 benzoyle dans une solution de 18 g d'amino-2 chloro-7 naphtyridine-1,8 dans 215 cm3 de pyridine anhydre en maintenant la température au voisinage de 20°C.

Le mélange réactionnel est agité encore pendant 1 heure à 20°C puis versé dans 1200 cm3 d'eau. Le précipité obtenu est séparé par filtration, lavé avec 300 cm3 d'eau et séché à l'air pour donner 32,5 g d'un solide fondant à 220°C. Par recristallisation de 15,5 g du produit ainsi obtenu, dans 400 cm3 d'acétonitrile,

on obtient 11,4 g de N- (chloro-7 naphtyridine-1,8 yl-2) acétoxy-4 benzamide fondant à 220°C.

Le chlorure d'acétoxy-4 benzoyle peut être préparé selon la méthode décrite par Von H. Schönenberger et coll., Arz. Forsch. *14*, 324 (1964).

Exemple 4

On opère d'une manière analogue à celle décrite à l'exemple 3, mais à partir de 40,5 g de chlorure d'acétoxy-3 benzoyle de 32 g d'amino-2 chloro-7 naphtyridine-1,8 et de 360 cm3 de pyridine anhydre. On obtient après précipitation du mélange réactionnel dans 2000 cm3 d'eau, lavage par 3 fois 500 cm3 d'eau et séchage, 58.8 g d'un solide cristallisé fondant à environ 195°C. Par recristallisation de 12 g du produit ainsi obtenu dans 350 cm3 d'acétonitrile, on obtient 7,5 g de N- (chloro-7 naphtyridine-1,8 yl-2) acétoxy-3 benzamide fondant à 207°C.

Le chlorure d'acétoxy-4 benzoyle peut être préparé selon la méthode décrite par Von H. Schönenberger et coll. Arz. Forsch. *14*, 324 (1964).

Exemple 5

On opère d'une manière analogue à celle décrite à l'exemple 3, mais à partir de 3,5 g d'amino-2 methoxy-7 naphtyridine, de 4,4 g de chlorure d'acétoxy-3 benzoyle et de 40 cm3 de pyridine anhydre. Le mélange réactionnel est versé dans 400 cm3 d'eau et le précipité obtenu est séparé par filtration puis lavé par 6 fois 100 cm3 d'eau et séché à l'air. Par recristallisation du produit ainsi obtenu dans 140 cm3 d'éthanol, on obtient 5,3 g de N-(méthoxy-7 naphtyridine-1,8 yl-2) acétoxy-3 benzamide fondant à 172°C.

L'amino-2 méthoxy-7 naphtyridine-1,8 peut être préparée selon la méthode décrit dans le brevet US 3 948 917.

Exemple 6

On opère d'une manière analogue à celle décrite à l'exemple 2, mais à partir de 20,7 g d'acide méthylthio-4 benzoïque, de 25,1 g de N,N'-carbonyldiimidazole et de 27,8 g d'amino-2 chloro-7 naphtyridine-1,8. Le produit obtenu après précipitation du mélange réactionnel dans l'eau et séchage est purifié par chromatographie sur une colonne de 40 mm de diamètre contenant 300 g de silice (0,06—0,20 mm), en éluant par un mélange (90/10 en volumes) de chlorure de méthylène et de méthylcyclohexane, et en recueillant des fractions de 100 cm3. Après concentration à sec des fractions 15 à 44, à 40°C sous pression réduite (4 kPa), on obtient 8,9 g d'un solide qui est recristallisé dans 550 cm3 d'acétate d'éthyle. On obtient ainsi 6,3 g de (N-chloro-7 naphtyridine-1,8 yl-2) méthylthio-4 benzamide fondant à 199°C.

Exemple 7

On opère d'une manière analogue à celle décrite à l'exemple 2 mais à partir de 10 g d'acide éthoxycarbonylamino-3 benzoïque, de 7,6 g de N,N'-carbonyldiimidazole et de 5,95 g d'amino-2 méthoxy-7 naphtyridine-1,8. Le mélange réactionnel est ensuite versé dans 1000 cm3 d'eau et le précipité formé est séparé par filtration, lavé par 10 cm3 d'eau et séché à l'air. Par recristallisation du produit ainsi obenu dans 100 cm3 d'acétate d'éthyle, on obtient 7,3 g de N-(méthoxy-7 naphtyridine-1,8 yl-2) éthoxycarbonylamino-3 benzamide fondant à 120°C.

L'acide éthoxycarbonylamino-3 benzoïque peut être préparé de la façon suivante:

On laisse agiter pendant 30 minutes à 5°C un mélange composé de 20 g d'acide amino-3 benzoïque, de 730 cm3 d'une solution aqueuse 1M de bicarbonate de sodium et de 14 cm3 de chloroformiate d'éthyle. On ajoute ensuite au mélange réactionnel 300 cm3 d'acide chlorhydrique 2,5N. Le précipité formé est séparé par filtration, lavé par 3 fois 200 cm3 d'eau et séché à 40°C sous pression réduite (0,07 kPa). On obtient ainsi 25 g d'acide éthoxycarbonylamino-3 benzoïque fondant à 198°C.

Exemple 8

On opère d'une manière analogue à celle décrite à l'exemple 2, mais à partir de 14,6 g d'acide diméthoxy-3,4 benzoïque de 12,9 g de N,N'-carbonyldiimidazole et de 8,9 g d'amino-2 chloro-7 naphtyridine-1,8, mais en chauffant 17 heures au reflux. Le produit obtenu après précipitation du mélange réactionnel dans 1000 cm3 d'eau et séchage, est purifié par recristallisation dans 250 cm3 d'acétonitrile. On obtient ainsi 8,5 g de N-(chloro-7 naphtyridine-1,8 yl-2) diméthoxy-3,4 benzamide fondant à 196°C.

Exemple 9

On opère d'une manière analogue à celle décrite à l'exemple 2, mais à partir de 14,56 g d'acide diméthoxy-3,5 benzoïque, de 12,9 g de N,N'-carbonyldiimidazole et de 8,9 g d'amino-2 chloro-7 naphtyridine-1,8, et en chauffant 20 heures au reflux.

Le produit obtenu après précipitation dans 2000 cm3 d'eau et séchage est purifié par recristallisation dans 900 cm3 d'acétonitrile. On obtient ainsi 10,75 g de N-(chloro-7 naphtyridine-1,8 yl-2) diméthoxy-3,5 benzamide fondant à 235°C.

Exemple 10

On opère d'une manière analogue à celle décrite à l'exemple 1, mais à partir de 7,4 g d'acide pyridinecarboxylique-1, de 9,7 g de N,N'-carbonyldiimidazole et de 8,1 g d'amino-2 chloro-7 naphtyridine-1,8, et en effectuant la réaction pendant 18 heures à une température voisine de 25°C. Le produit obtenu après filtration, lavage avec 60 cm3 de tétrahydrofuranne et 120 cm3 d'eau, puis séchange, est mis en suspension dans 120 cm3 d'éthanol et agité pendant 1 heure à 25°C. Le produit insoluble est séparé par filtration et séché à 45°C sous pression réduite (0,07 kPa). On obtient ainsi 8,2 g de N-(chloro-7 naphtyridine-1,8 yl-2) pyridinecarboxamide-2 fondant à 319°C.

Exemple 11

On opère d'une manière analogue à celle décrite à l'exemple 2, mais à partir de 6,2 g d'acide pyridinecarboxylique-4 de 8,1 g de N,N'-carbonyldiimidazole et de 9 g d'amino-2 chloro-7 naphtyridine-1,8. Le produit obtenu après précipitation dans 500 cm3 d'eau, lavage avec 50 cm3 de tétrahydrofuranne, puis lavage avec 200 cm3 d'eau et séchage (9,8 g; F = environ 220°C) est purifié par cristallisation dans 850 cm3 d'acétonitrile. On obtient ainsi 5.2 g de N-(chloro-7 naphtyridine-1,8 yl-2) pyridinecarboxamide-4 fondant à 228°C.

Exemple 12

On opère d'une manière analogue à celle décrite à l'exemple 1, mais à partir de 7,5 g d'acide pyrazinecarboxylique-2 de 9,7 g de N,N'-carbonyldiimidazole et de 10,8 g d'amino-2 chloro-7 naphtyridine-1,8. Le produit insoluble est séparé par filtration, lavé 3 fois 50 cm3 de tétrahydrofuranne puis par 300 cm3 d'eau et séché à l'air. Le produit est mis en suspension dans 100 cm3 d'acétonitrile, agité pendant 2 heure à 25°C est séparé par filtration puis séché à 50°C sous pression réduite (0,07 kPa). On obtient ainsi 11,75 g de N-(chloro-7 naphtyridine-1,8 yl-2) pyrazinecarboxamide-2 fondant à 308°C.

Exemple 13

On ajoute en 10 minutes à une température de 25°C une solution de 14,3 g de chlorure de dihydro-5,6-dithiinne-1,4 carbonyle-2 dans 50 cm3 de pyridine anhydre à une solution de 28,6 g d'amino-2 chloro-7 naphtyridine-1,8 dans 300 cm3 de pyridine anhydre. Après 3 heures d'agitation à une température de 25°C, l'insoluble est séparé par filtration, lavé par 2 fois 15 cm3 de pyridine puis 20 cm3 d'oxyde d'isopropyle. Le filtrat est concentré à sec à 60°C sous pression réduite (4 kPa) et le résidu est repris par 200 cm3 d'eau. Le solide cristallisé obtenu est séparé par filtration, lavé par 200 cm3 d'eau et dissous dans 500 cm3 d'un mélange chloroforme-méthanol (90/10 en volume). La solution obtenue est lavée par 100 cm3 d'une solution 0.5N d'acide chlorhydrique puis 3 fois 100 cm3 d'eau, séchée sur sulfate de sodium et concentrée à sec à 60°C sous pression réduite (4 kPa).

Le solide obtenu est purifié par chromatographie "Flash" sous 40 kPa sur une colonne de 63 mm de diamètre contenant 500 g de silice (0,04—0,06 mm) en éluant par du chloroforme et en recueillant des fractions de 300 cm3. Après concentration à sec des fractions 2 à 8 à 60°C sous pression réduite (4 kPa)., on obtient 15 g d'un solide qui est agité en suspension dans 325 cm3 d'acétonitrile pendant 1 heure à 0°C. Le produit insoluble est séparé par filtration, lavé par 2 fois 30 cm3 d'acétonitrile et séché à l'air. On obtient 11,9 g de N-(chloro-7 naphtyridine-1,8 yl-2) dihydro-5,6 dithiinne-1,4 carboxamide-2 fondant à 237°C.

Le chlorure de dihydro-5,6 dithiinne-1,4 carbonyle-2 peut être préparé de la façon suivante:

Une suspension de 13,1 g d'acide dihydro-5,6 dithiinne-1,4 carboxylique-2 dans 60 cm3 de chlorure de thionyle est chauffée progressivement jusqu'au reflux. Après 30 minutes à reflux la solution obtenue est concentrée à sec à 50°C sous pression réduite (4 kPa) et le résidu huileux obtenu est lavé par 2 fois 100 cm3 de cyclohexane. L'uile décantée est séchée à 50°C sous pression réduite (4 kPa). On obtient ainsi 15,8 g de chlorure de dihydro-5,6 dithiinne-1,4 carbonyle-2 qui est utilisé tel quel dans l'opération suivant.

Exemple 14

On opère d'une manière analogue à celle décrite à l'exemple 2, mais à partir de 10,9 g d'acide dihydro-5,6 dithiinne-1,4 carboxylique-2 de 10,9 g de N,N'-carbonyldiimidazole et de 11,9 g d'amino-2 phénoxy-7 naphtyridine-1,8 et en chauffant pendant 18 heures à reflux. Le produit obtenu après précipitation dans l'eau, lavage à l'eau et séchage (18 g; F = environ 240°C) est purifié par recristallisation dans 150 cm3 de diméthylformamide. On obtient ainsi 15 g de N-(phénoxy-7 naphtyridine-1,8 yl-2) dithiinne-1,4 dihydro-5,6 carboxamide-2 fondant à 245°C.

L'acide dihydro-5,6 dithiinne-1,4 carboxylique-2 peut être préparé selon K. G. GUNDERMANN et coll., Ber *95,* 2076 (1962).

Exemple 15

On opère d'une manière analogue à celle décrite à l'exemple 2, mais à partir de 7,3 g d'acide dihydro-5,6 oxathiine-1,4 carboxylique-2, de 9,7 g de N,N'-carbonyldiimidazole et de 7,2 g d'amino-2 chloro-7 naphtyridine-1,8. Le produit obtenu précipitation dans l'eau est purifié par recristallisation dans 60 cm3 de diméthylformamide. On obtient ainsi 5,9 g de N-(chloro-7 naphtyridine-1,8 yl-2) dihydro-5,6 oxathiine-1,4-carboxamide-2 fondant à 270°C.

L'acide dihydro-5,6 oxathiine-1,4 carboxylique-2 peut être préparé de la manière suivante:

A une solution de 15,6 g de mercapto-2 éthanol dans 50 cm3 d'éthanol, on ajoute en 20 minutes, 39 g de bromopyruvate d'éthyle. Le mélange réactionnel est agité pendant 2 heures, puis concentré à sec sous pression réduite (4 kPa). Le produit huileux obtenu (37 g) est ajouté à une mélange de 30 cm3 de soude 10N

et 170 cm3 d'éthanol. Après 2 heures de chauffage au reflux, le mélange réactionnel est concentré à sec sous pression réduite (4 kPa), puis dissous dans 110 cm3 d'eau distillée. Le précipité obtenu après neutralisation par l'acide chlorhydrique concentré (d = 1,19) est séparé par filtration, lavé par 3 fois 50 cm3 d'eau distillée et séché à l'air. On obtient 10,8 g d'acide dihydro-5,6 oxathiine-1,4 carboxylique-2 fondant à 144°C.

Exemple 16

On opère d'une manière analogue à celle décrite à l'exemple 2, mais à partir de 4,6 g d'acide dithiole-1,3 carboxylique-4 de 7,5 g de N,N'-carbonyldiimidazole et de 4,2 g d'amino-2 chloro-7 naphtyridine-1,8. Le produit obtenu par précipitation dans l'eau (4,8 g) est repris par 300 cm3 d'acétonitrile bouillant, l'insoluble est éliminé par filtration et le filtrat est versé dans 500 cm3 d'eau. Le précipité obtenu est purifié par recristallisation dans 650 cm3 d'un mélange acétonitrile-eau (5/2 en volumes). On obtient ainsi 2,8 g de N-(chloro-7 naphtyridine-1,8 yl-2) dithiole-1,3 carboxamide-4 fondant à 235°C.

L'acide dithiole-1,3 carboxylique-4 peut être préparé par application de la méthode décrite par: C. U. Pittman Jr et coll., J.C.S. Chem. Comm., 960 (1975).

Exemple 17

On opère d'une manière analogue à celle décrite à l'exemple 2, mais à partir de 4,8 g d'acide thiazolecarboxylique-4 de 7,5 g de N,N'-carbonyldiimidazole et de 4,8 g d'amino-2 chloro-7 naphtyridine-1,8. Le produit obtenu par précipitation dans l'eau est purifié par recristallisation dans 350 cm3 de diméthylformamide. On obtient ainsi 6 g de N-(chloro-7 naphtyridine-1,8 yl-2) thiazolecarboxamide-4 fondant à 325°C.

L'acide thiazolecarboxylique-4 peut être préparé par application de la méthode décrite par: H. Erlenmeyer et coll., Helv. Chim. Acta, *25*, 1073 (1942).

Exemple 18

On opère d'une manière analogue à celle décrite à l'exemple 2, mais à partir de 4,5 g d'acide thiazolecarboxylique-5 de 6,8 g de N,N'-carbonyldiimidazole et de 5 g d'amino-2 chloro-7 naphtyridine-1,8. Le produit obtenu par précipitation dans l'eau est purifié par recristallisation dans 120 cm3 d'un mélange diméthylformamide-acétonitrile (5/5 volumes). On obtient ainsi 5,5 g de N-(chloro-7 naphtyridine-1,8 yl-2) thiazolecarboxamide-5 fondant à 292°C.

L'acide thiazolecarboxylique-5 peut être préparé par application de la méthode décrite par H. Erlenmeyer et coll., Helv. Chim. Acta, *20*, 204 (1937).

Exemple 19

On opère d'une manière analogue à celle décrite à l'exemple 2, mais à partir de 9,5 g d'acide chloro-5 thiophènecarboxylique-2, de 9,5 g de N,N'-carbonyldiimidazole et de 8 g d'amino-2 chloro-7 naphtyridine-1,8. Le produit obtenu par précipitation dans l'eau est purifié par recristallisation dans 700 cm3 de propanol. On obtient ainsi 8,3 g de N-(chloro-7 naphtyridine-1,8 yl-2) chloro-5 thiophènecarboxamide-2 fondant à 275°C.

L'acide chloro-5 thiophènecarboxylique-2 peut être préparé par application de la méthode décrite par L. Gatterman et coll., Chem. Ber., *19* 688 (1886).

Exemple 20

On opère d'une manière analogue à celle décrite à l'exemple 2, mais à partir de 5,6 g d'acide méthylthio-5 thiophènecarboxylique-2, de 5,2 g de N,N'-carbonyldiimidazole et de 4,3 g d'amino-2 chloro-7 naphtyridine-1,8. Le produit obtenu par précipitation dans l'eau est purifié par recristallisation dans 200 cm3 d'un mélange diméthylformamide-méthanol (5/5 en volumes). On obtient ainsi 6,1 g de N-(chloro-7 naphtyridine-1,8 yl-2) méthylthio-5 thiophènecarboxamide-2 fondant à 235°C.

L'acide méthylthio-5 thiophènecarboxylique-5 peut être préparé par application de la méthode décrite par J. Cymerman-Craig et coll., J. Chem. Soc., 237 (1954).

Exemple 21

On opère d'une manière analogue à celle décrite à l'exemple 2, mais à partir de 2,9 g d'acide bromo-5 furannecarboxylique-2 de 2,4 g de N,N'-carbonyldiimidazole et de 1,8 g d'amino-2 chloro-7 naphtyridine-1,8. Le produit obtenu par précipitation dans l'eau est purifié par recristallisation dans 100 cm3 d'un mélange diméthylformamide-méthanol (5/5 en volumes). On obtient ainsi 2,6 g de N-(chloro-7 naphtyridine-1,8 yl-2) bromo-5 furannecarboxamide-2 fondant à 285°C.

Exemple 22

On ajoute en 30 minutes en agitant, 42 g de chlorure d'acryloyle à une solution de 54 g d'amino-2 chloro-7 naphtyridine-1,8 et de 115 g de triéthylamine dans 1875 cm3 de chlorure de méthylène. La soluton obtenue et chauffée pendant 1 heure à reflux puis versée dans 5000 cm d'oxyde d'isopropyle. Le précipité obtenu est filtré, lavé par 3 fois 200 cm3 d'oxyde d'isopropyle puis 3 fois 200 cm3 d'eau à 40°C et séché à l'air. Le produit brut obtenu est purifié par chromatographie "Flash" (sous 50 kPa) sur une colonne de 60

mm de diamètre contenant 500 g de silice (0,04—0,06 mm) en éluant par un mélange (99/1 en volumes) de chlorure de méthylène et de méthanol et en recueillant des fractions de 100 cm3. Après concentration à sec des fractions 5 à 10 à 40°C sous pression réduite (4 pKa), on obtient 5,85 g de N-(chloro-7 naphtyridine-1,8 yl-2) arcrylamide fondant à 200°C.

Exemple 23

A une solution de 9 g d'amino-2 chloro-7 naphtyridine-1,8 et 19 g de triéthylamine dans 300 cm3 de chlorure de méthylène, on ajoute en 20 minutes, 9 g de chlorure de méthacryloyle en solution dans 30 cm3 de chlorure de méthylène. Aprés 30 minutes de reflux, la solution obtenue est lavée par 150 cm3 d'acide chlorhydrique 0,5N et 2 fois 150 cm3 d'eau distillée, puis concentré à sec sous pression réduite (4 kPa). Le solide obtenu (13,5 g) est dissous dans 350 cm3 de méthanol bouillant. Après 3 heures de refroidissement à 4°C, le solide cristallisé est séparé par filtration, lavé par 2 fois 30 cm3 de méthanol et séché à l'air. Le produit insolé (9 g; F = 195°C) est alors ajouté en 5 minutes à 19 cm3 d'acide sulfurique concentré (d = 1,83). Le mélange réactionnel est chauffé 10 minutes à 60°C, agité 1 heure à 25°C, puis versé dans 200 cm3 d'eau distillée. Le précipité formé est séparé par filtration, lavé par 5 fois 20 cm3 d'eau distillée et recristallisé dans 70 cm3 d'acétonitrile. On obtient ainsi 5,4 g de N-(chloro-7 naphtyridine-1,8 yl-2) méthacrylamide fondant à 182°C.

Exemple 24

On opère d'une manière analogue à celle décrite à l'exemple 2, mais à partir de 18,24 g d'acide méthoxy-4 benzoïque, de 19,44 de N,N'-carbonyldiimidazole et de 20,6 g d'amino-2 (chloro-3 phénoxy-7) naphtyridine 1,8, en chauffant 2 heures au reflux.

Le mélange réactionnel est versé dans 1000 cm3 d'eau distillée et 1000 cm3 de chlorure de méthylène. La phase aqueuse est extraite par 250 cm3 de chlorure de méthylène. Les extraits organiques sont réunis puis lavés avec 250 cm3 d'eau distillée, séchés sur sulfate de magnésium et concentrés à sec à 50°C sous pression réduite (4 kPa). Le produit brut obtenu est purifié par recristallisation dans 700 cm3 d'acétonitrile. On obtient ainsi 20 g de N-[(chloro-3 phénoxy)-7 naphtyridine-1,8 yl-2] méthoxy-4 benzamide fondant à 182°C.

L'amino-2 (chloro-3 phénoxy)-7 naphtyridine-1,8 peut être préparée de la façon suivante:

On ajoute 17,95 g d'amino-2-chloro naphtyridine-1,8 à un mélange préchauffé à 60°C de 51,4 g de chloro-3 phénol et de 13,2 g de potasse en pastilles à 85%. Le mélange réactionnel est chauffé à 120°C pendant 3 heures. Le produit est séparé par filtration, lavé à l'eau jusqu'à un pH d'environ 7 puis séché à l'air. On obtient 20,6 g d'amino-2 (chloro-3 phenoxy)-7 naphtyridine-1,8 fondant à 166°C.

Exemple 25

On opère d'une manière analogue à celle décrite à l'exemple 2, mais à partir de 8,5 g d'acide méthoxy-4 benzoïque, de 9 g de N,N'-carbonyldiimidazole et de 10,7 g d'amino-2 (dichoro-3,4 phénoxy)-7 naphtyridine-1,8. Le produit obtenu par précipitation dans l'eau est purifié par chromatographie "flash" (sous 30 kPa) sur une colonne de 40 mm de diamètre contenant 200 g de silice (0,04—0,06 mm) en éluant par un mélange (9/1 en volumes) de chlorure de méthylène et de méthanol et en recueillant des fractions de 100 cm3. Après concentration à sec des fractions 6 à 12 à 40°C sous pression réduite (4 kPa), on obtient 6,35 g d'un solide qui est recristallisé dans 160 cm3 d'acétonitrile. On obtient ainsi 5,55 g de N-[(dichloro-3,4 phénoxy)-7 naphtyridine-1,8 yl-2] méthoxy-4 benzamide fondant à 180°C.

L'amino-2 (dichloro-3,4 phénoxy)-7 naphtyridine-1,8 peut être préparée de la façon suivante:

On opère d'une manière analogue à celle décrité à l'exemple 24 mais à partir de 17,95 g d'amino-2 chloro-7 naphtyridine-1,8, de 65,2 g de dichloro-3,4 phénol et de 13,2 g de potasse en pastilles à 85%, en chauffant à 125°C pendant 22 heures. Par recristallisation du produit brut obtenu dans 1500 cm3 d'acétonitrile, on obtient 18 g d'amino-2 (dichloro-3,4 phénoxy)-7 naphtyridine-1,8 fondant à 206°C.

Exemple 26

On opère d'une manière analogue à celle décrite à l'exemple 1, mais à partir de 8,2 g d'acide méthoxy-4 benzoïque, de 8,7 g de N,N'-carbonyldiimidazole et de 6,9 g d'amino-2 cyano-7 naphtyridine-1,8 et de en chauffant 4 heures à reflux. Le produit insoluble est séparé par filtration et le filtrat est versé dans 1000 cm3 d'eau. Le précipité résultant est filtré, lavé par 3 fois 75 cm3 d'eau et séché à l'aire. Le solide obtenu est purifié par chromatographie "flash" (sous 30 kPa) sur une colonne de 40 mm de diamètre contenant 170 g de silice (0,04—0,06 mm) en éluant par du chlorure de méthylène pur et en recueillant des fractions de 50 cm3. Après concentration à sec des fractions 5 à 27 à 40°C sous pression réduite (4 kPa), le produit obtenu est purifié par recristallisation dans 160 cm3 de dioxanne. On obtient ainsi 1,4 g de N-(cyano-7 naphtyridine-1,8 yl-2) méthoxy-4 benzamide fondant à 283°C.

L'amino-2 cyano-7 naphtyridine-1,8 peut être préparée selon la méthode décrite dans le brevet belge 815 019.

Exemple 27

On opère d'une manière analogue à celle décrite à l'exemple 2, mais à partir de 2,2 g d'acide méthoxy-6 pyridazinecarboxylique-3 de 2,3 g de N,N'-carbonyldiimidazole et de 2,7 g d'amino-2 phénoxy-7

naphtyridine-1,8. Le produit obtenu par précipitation dans l'eau et purifié par recristallisation dans 300 cm3 d'acetonitrile. On obtient ainsi 3 g de N-(phénoxy-7 naphtyridine-1,8 yl-2) méthoxy-6 pyridazinecarboxamide-3 fondant à 95°C.

L'acide méthoxy-6 pyridazinecarboxylique-3 peut être préparé par application de la méthode décrite par A. Pollak, et coll., Monatsh., Chem., *106*, 473 (1975).

Exemple 28

On chauffe à reflux pendant 2 heures une solution de 5 g de N-(chloro-7 naphtyridine-1,8 yl-2) méthoxy-4 benzamide dans 60 cm3 d'acide acétique et 15 cm3 d'anhydride acétique. La solution est refroidie et versée dans 750 cm3 d'eau, la suspension obtenue est alcalinisée à pH 10 par de la soude 10N et le solide est séparé par filtration. Après lavage à l'eau et à l'acétone puis séchage à 50°C sous pression réduite (0,07 kPa), on obtient 4 g de N-(hydroxy-7 naphtyridine-1,8 yl-2) méthoxy-4 benzamide fondant à environ 346°C.

Le N-(chloro-7 naphtyridine-1,8 yl-2) méthoxy-4 benzamide peut être préparé de la manière suivant:

A une solution de 4,6 g d'acide méthoxy-4 benzoïque dans 30 cm3 de tétrahydrofuranne anhydre, on ajoute 4,9 g N,N'-carbonyldiimidazole. On observe un dégagement gazeux immédiat. Le mélange est agité 1 heure à une température voisine de 20°C, jusqu'à la fin du dégagement gazeux. On ajoute ensuite 3,6 g d'amino-2 chloro-7 naphtyridine-1,8 et on chauffe à reflux pendant 18 heures.

Le produit obtenu par précipitation dans l'eau (4,3 g; F = 208°C) est dissous dans 660 cm3 d'acétonitrile bouillant. Après 3 heures de refroidissement à 4°C, le solide cristallisé est séparé par filtration, lavé par 3 fois 20 cm3 d'acétonitrile et séché à 50°C sous pression réduite (0,07 kPa). On obtient 3,5 g de N-(chloro-7 naphtyridine-1,8 yl-2) méthoxy-4 benzamide fondant à 208°C.

La présente invention concerne également les compositions pharmaceutiques qui contiennent les nouveaux produits de formule générale (I) à l'état pur ou en association avec un adjuvant, un diluant et/ou enrobage compatibles en pharmaceutiquement acceptables. Ces compositions peuvent être employées par voie orale, rectale, parentérale ou percutanée.

Comme compositions solides pour administration orale peuvent être utilisés des comprimés, des pilules, des poudres (généralement dans des capsules de gélatine) ou des granulés. Dans ces compositions, le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants insertes tels que l'eau ou l'huile de paraffine. Ces compositions peuvent également comprendre des substances autres que les diluants, par exemple produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer la propylèneglycol, un polyéthylèneglycol, des huiles végétales, en particulier l'huile d'olive ou des esters organiques injectables, par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparés sous forme de compositions solides stériles qui puevent être dissoutes au moment de l'emploi dans l'eau stérile ou tout autre milieu stérile injectable.

Les compositions pour administration rectale sont des suppositoires qui peuvent contenir, outre le produit actif, des excipients tels que la beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule favorisant le migration percutanée.

Les compositions selon l'invention sont particulièrement utiles en thérapeutique humaine pour leur action anxiolytique, hypnotique et myorelaxante.

En thérapeutique humaine, les doses dépendent de l'effet recherché et de la durée du traitement; elles sont généralement comprises entre 10 et 500 mg par jour par voie orale pour un adulte.

D'une façon générale, le médecin déterminera la posologie qui'il estime la plus appropriée en fonction de l'âge du poids et de tous autres facteurs propres au sujet à traiter.

Les exemples suivants, donnés à titre non limitatif, illustrent une composition selon l'invention.

Exemple A

On prépare selon la technique habituelle des comprimés dosés à 10 mg de produit actif ayant la composition suivante:

| | |
|---|---|
| N-(chloro-7 naphtyridine-1,8 yl-2) acétoxy-3 benzamide | 0,010 g |
| amidon | 0,200 g |
| silice précipitée | 0,036 g |
| stéarate de magnésium | 0,004 g |

Exemple B

On prépare selon la technique habituelle des comprimés dosés à 10 mg de produit actif ayant la composition suivante:

| | |
|---|---|
| N-(chloro-7 naphtyridine-1,8 yl-2) dithiole-1,3 carboxamide-4 | 0,010 g |
| amidon | 0,200 g |
| silice précipitée | 0,036 g |
| stéarate de magnésium | 0,004 g |

Exemple C

On prépare selon la technique habituelle des comprimés dosés à 10 mg de produit actif ayant la composition suivante:

| | |
|---|---|
| N-(cyano-7 naphtyridine-1,8 yl-2) méthoxy-4-benzamide | 0,010 g |
| amidon | 0,200 g |
| silice précipitée | 0,036 g |
| stéarate de magnésium | 0,004 g |

**Revendications pour les Etats contractants: BE CH DE FR GB IT LI LU NL SE**

1. Un nouvel amide substitué caractérisé en ce qu'il répond à la formule générale:

R—CONH—Het

dans laquelle:

soit le symbole R représente un radical phényle substitué par un radical acyloxy, alcoylthio ou alcoyloxycarbonylamino, par un atome de chlore en position -2 ou -4 ou par deux radicaux alcoyloxy, ou représente un radical hétérocyclyle choisi parmi pyridyle-2, pyridyle-4, pyrazinyle, dihydro-5,6 dithiinnyle-2, dihydro-5,6 oxathinnyle-2, dithiolyle-1,3, thiazolyle ou, thiényle ou furyle substitués par un atome d'halogène ou par un radical alcoylthio ou R représente un radical alcényle contenant 2 à 4 atomes de carbone et dont la double liaison se trouve en α par rapport au groupement carbonyle de la fonction amide, et le symbole Het représente un radical naphtyridine-1,8 yle-2 substitué en position-7 par une atome d'halogène ou par un radical alcoyloxy, phénoxy, chloro-3 phénoxy, dichlorophénoxy, hydroxy ou cyano,

soit le symbole R représente un radical méthoxyphényle et le symbole Het représente un radical naphtyridine-1,8 yle-2 substitué en position -7 par un radical chloro-3 phénoxy, dichlorophénoxy, hydroxy ou cyano,

soit le symbole R représente un radical méthoxy pyridazinyle-3 et le symbole Het représente un radical naphtyridine-1,8 yle-2 substitué en position -7 par un radical phénoxy,

étant entendu que les radicaux alcoyle et acyle cités ci-dessus contiennent 1 à 4 atomes de carbone en chaîne droite ou ramifiée.

2. Un nouvel amide selon la revendication 1 caractérisé en ce que soit le symbole R représente un radical phényle substitué par un radical acyloxy, alcoylthio ou alcoyloxycarbonylamino, par un atome de chlore en position -2 ou -4 ou par 2 radicaux alcoyloxy, ou représente un radical hétérocyclyle choisi parmi pyridyle-2, pyridyle-4, pyrazinyle, dihydro-5,6, dithiinnyle-2, dihydro-5,6 oxathinnyle-2, dithiolyle-1,3, thiazolyle, thiényle-2 ou furyle-2 substitués position -5 par un atome d'halogène ou alcoylthio-5 thiényle-2 ou R représente un radical alcényle contenant 2 à 4 atomes de carbone et dont la double liaison se trouve en α par rapport au groupement carbonyle de la fonction amide, et le symbole Het représente un radical naphtyridine-1,8 yle-2 subsitué en position -7 par un atom d'halogène ou par un radical alcoyloxy ou phénoxy,

soit le symbole R représente un radical méthoxyphényle et le symbole Het représente un radical naphtyridine-1,8 yle-2 substitué en position -7 par un radical chloro-3 phénoxy, dichloro-3,4 phénoxy, hydroxy ou cyano,

soit le symbole R représente un radical méthoxy-6 pyridazinyle-3 et le symbole Het représente un radical naphtyridine-1,8 yle-2 substitué en position -7 par un radical phénoxy,

étant entendu que les radicaux alcoyle et acyle cités ci-dessus contiennent 1 à 3 atomes de carbone en chaîne droite ou ramifiée.

3. Un nouvel amide selon la revendication 1 caractérisé en ce que

soit le symbole R représente un radical phényle substitué par un radical acyloxy ou représente un radical hétérocyclyle choisi parmi dihydro-5,6 dithiinnyle-2, dithiolyle-1,3, ou thiazolyle-4 et le symbole Het

représente un radical naphtyridine-1,8 yle-2 substitué en position-7 par une atome d'halogène ou par un radical méthoxy,

soit le symbole R représente un radical méthoxy-4 phényle et le symbole Het représente un radical naphtyridine-1,8 yle-2 substitué en position -7 par un radical chloro-3 phénoxy ou cyano.

4. Le N-(choro-7 naphtyridine-1,8 yl-2) acétoxy-4 benzamide.

5. Le N-(choro-7 naphtyridine-1,8 yl-2) acétoxy-3 benzamide.

6. Le N-(méthoxy-7 naphtyridine-1,8 yl-2) acétoxy-3 benzamide.

7. Le N-(chloro-7 naphtyridine-1,8 yl-2) dithiole-1,3 carboxamide-4.

8. Le N-(cyano-7 naphtyridine-1,8 yl-2) méthoxy-4 benzamide.

9. Un procédé de préparation d'un amide substitué selon la revendication 1, caractérisé en ce que l'on fait agir un acide de formule générale:

R—COOH

dans laquelle R est défini comme dans la revendication 1, ou un dérivé réactif de cet acide, sur une amine de formule générale:

$Het—NH_2$

dans laquelle Het est défini comme dans la revendication 1.

10. Un procédé selon la revendication 2, caractérisé en ce quel l'on fait réagir un dérivé réactif de l'acide, choisi parmi l'anhydride, un anhydre mixte, un halogdénure d'acide ou un ester.

11. Un procédé de préparation d'un amide substitué selon la revendication 1, pur lequel le symbole Het contient un substituant hydroxy, caractérisé en ce que l'on traite en milieu acide l'amide substitué correspondant, défini selon la revendication 1, pour lequel Het est un radical naphtyridine-1,8 yle-2 substitué en position-7 par un atomes d'halogène, puis hydrolyse le produit obtenu.

12. Composition pharmaceutique caractérisée en ce qu'elle contient au moins un amide selon la revendication 1, à l'état pur, ou en association avec un plusieurs diluants ou adjuvants compatibles et pharmaceutiquement acceptables.

**Revendication pour les Etats contractants: AT GR ES**

Procédé de préparation d'un nouvel amide substitué caractérisé en ce qu'il répond à la formule générale:

R—CONH—Het (I)

dans laquelle:

soit le symbole R représente un radical phényle substitué par un radical acyloxy, alcoylthio ou alcoyloxycarbonylamino, par un atome de chlore en position -2 ou -4 ou par deux radicaux alcoyloxy, ou représente un radical hétérocyclyle choisi parmi pyridyle-2, pyridyle-4, pyrazinyle, dihydro-5,6 dithiinnyle-2, dihydro-5,6 oxathinnyle-2, dithiolyle-1,3, thiazolyle ou, thiényle ou furyle substitués par un atome d'halogène ou par un radical alcoylthio ou R représente un radical alcényle contenant 2 à 4 atomes de carbone et dont la double liaison se trouve en α par rapport au groupement carbonyle de la fonction amide, et le symbole Het représente un radical naphtyridine-1,8 yle-2 substitué en position-7 par une atome d'halogène ou par un radical alcoyloxy, phénoxy, chloro-3 phénoxy, dichlorophénoxy, hydroxy ou cyano,

soit le symbole R représente un radical méthoxyphényle et le symbole Het représente un radical naphtyridine-1,8 yle-2 substitué en position -7 par un radical chloro-3 phénoxy, dichlorophénoxy, hydroxy ou cyano,

soit le symbole R représente un radical méthoxy pyridazinyle-3 et le symbole Het représente un radical naphtyridine-1,8 yle-2 substitué en position -7 par un radical phénoxy,

étant entendu que les radicaux alcoyle et acyle cités ci-dessus contiennent 1 à 4 atomes de carbone en chaîne droite ou ramifiée, caracterisé en ce que l'on fait agir un acide de formule générale:

R—COOH

dans laquelle R est défini comme ci-dessus, ou un dérivé réactif de cet acide, sur une amine de formule générale:

$Het—NH_2$

dans laquelle Het est défini comme ci-dessus, ou bien pour la préparation d'un amide substitué pour lequel le symbole Het contient un substituant hydroxy, caractérisé en ce que l'on traite en milieu acide l'amide substitué correspondant, pour lequel Het est un radical naphtyridine-1,8 yle-2 substitué en position-7 par un atome d'halogène, puis hydrolyse le produit obtenu.

**Patentansprüche für die Vertragsstaaten: BE CH DE FR GB IT LI LU NL SE**

1. Ein neues substituirtes Amid, dadurch gekennzeichnet, daß es der allgemeinen Formel

R—CONH—Het

entspricht, worin:

entweder das Symbol R einen Phenylrest bedeutet, substituiert durch einen Acyloxy-, Alkylthio- oder Alkyloxycarbonylaminorest, durch ein Chloratom in 2- oder 4-Stellung oder durch zwei Alkyloxyreste oder einen heterocyclischen Rest bedeutet, ausgewählt aus Pyridyl-2, Pyridyl-4, Pyrazinyl, 5,6-Dihydro-dithiinyl-2, 5,6-Dihydro-oxathiinyl-2, 1,3-Dithiolyl, Thiazolyl, oder Thienyl oder Furyl substituiert durch ein Halogenatom oder durch einen Alkylthiorest oder R bedeutet einen Alkenylrest mit 2 bis 4 Kohlenstoffatomen und dessen Doppelbindung sich in α-Stellung in bezug auf die Carbonylgruppe der Amidfunktion befindet, und das Symbol Het bedeutet einen, 1,8-Naphthyridin-2-yl-Rest substituiert in 7-Stellung durch ein Halogenatom oder einen Alkyloxy-, Phenoxy-, 3-Chlorphenoxy-, Dichlorphenoxy-, Hydroxy- oder Cyanorest,

oder das Symbol R bedeutet einen Methoxyphenylrest und das Symbol Het bedeutet einen-1,8-Naphthyridin-2-yl-Rest substituiert in 7-Stellung durch einen 3-Chlorphenoxy-, Dichlorphenoxy-, Hydroxy- oder Cyanorest,

oder das Symbol R bedeutet einen 3-Methoxy-pyridazinylrest und das Symbol Het bedeutet einen 1,8-Naphthyridin-2-yl-Rest substituiert in 7-Stellung durch einen Phenoxyrest,

wobei die oben erwähnten Alkyl- und Acylreste 1 bis Kohlenstoffatome in gerader oder verzweigter Kette enthalten.

2. Ein neues Amid gemaß Anspruch 1, dadurch gekennzeichnet, daß das Symbol R einen Phenylrest bedeutet, substituiert durch einen Acyloxy-, Alkylthio- oder Alkyloxycarbonylamino-Rest, durch ein Chloratom in 2- der 4-Stellung oder durch zwei Alkyloxyreste oder einen heterocyclischen Rest bedeutet, ausgewählt unter Pyridyl-2, Pyridyl-4, Pyrazinyl, 5,6-Dihydro-dithiinyl-2, 5,6-Dihydro-oxathiinyl-2, 1,3-Dithiolyl, Thiazolyl, 2-Thienyl oder 2-Furyl substituiert in 5-Stellung durch ein Halogenatom oder 5-Alkylthio-2-thienyl oder R bedeutet einen Alkenylrest mit 2 bis 4 Kohlenstoffatomen, dessen Doppelbindung sich in α-Stellung in bezug auf die Carbonylgruppe der Amidfunktion befindet, und das Symbol Het bedeutet einen, 1,8-Naphthyridin-2-yl-Rest substituiert in 7-Stellung durch ein Halogenatom oder durch einen Alkyloxy- oder Phenoxyrest,

oder das Symbol R bedeutet einen Methoxyphenylrest und das Symbol Het bedeutet einen 1,8-Naphthyridin-2-yl-Rest substituiert in 7-Stellung durch einen 3-Chlor-phenoxy-, 3,4-Dichlor-phenoxy-, Hydroxy- oder Cyanorest,

oder das Symbol R bedeutet einen 6-Methoxy-3-pyridazinylrest und das Symbol Het bedeutet einen 1,8-Naphthyridin-2-yl-Rest substituiert in 7-Stellung durch einen Phenoxyrest, wobei die oben erwähnten Alkyl- und Acylreste 1 bis 3 Kohlenstoffatome in gerader oder verzweigter Kette enthalten.

3. Ein neues Amid gemäß Anspruch 1, dadurch gekennzeichnet, daß

entweder das Symbol R einen Phenylrest bedeutet substituiert durch einen Acyloxyrest oder einen heterocyclischen Rest bedeutet ausgewählt unter 5,6-Dihydro-dithiinyl-2, 1,3-Dithiolyl oder Thiazolyl-4 und das Symbol Het bedeutet einen 1,8-Naphthyridin-2-yl-Rest substituiert in 7-Stellung durch ein Halogenatom oder durch einen Methoxyrest,

oder das Symbol R bedeutet einen 4-Methoxy-phenyl-Rest und das Symbol Het bedeutet einen 1,8-Naphthyridin-2-yl-Rest substituiert in 7-Stellung durch einen 3-Chlor-phenoxy- oder Cyanorest.

4. N-(7-Chlor-1,8-naphthyridin-2-yl)-4-acetoxy-benzamid.

5. N-(7-Chlor-1,8-naphthyridin-2-yl)-3-acetoxy-benzamid.

6. N-(7-Methoxy-1,8-naphthyridin-2-yl)-3-acetoxy-benzamid.

7. N-(7-Chlor-1,8-naphthyridin-2-yl)-1,3-dithiol-4-carboxamid.

8. N-(7-Cyano-1,8-naphthyridin-2-yl)-4-methoxy-benzamid.

9. Verfahren zur Herstellung eines substituierten Amids gemäß Anspruch 1, dadurch gekennzeichnet, daß man eine Säure der allgemeinen Formel

R—COOH

worin R wie in Anspruch 1 definiert ist, oder ein reaktives Derivat dieser Säure auf ein Amin der allgemeinen Formel

Het —$NH_2$

worin Het wie in Anspruch 1 definiert ist, einwirken läßt.

10. Verfahren gemäß Anspruch 2, dadurch gekennzeichnet, daß man ein reaktives Derivat der Säure, ausgewählt unter dem Anhydrid, einem gemischten Anhydrid, einem Säurehalogenid oder einem Ester, reagieren läßt.

11. Verfahren zur Herstellung eines substituierten Amids gemäß Anspruch 1, wobei das Symbol Het einen Hydroxysubstituenten enthält, dadurch gekennzeichnet, daß man das entsprechende substituierte

Amid, definiert gemäß Anspruch 1, wobei Het ein 1,8-Naphthyridin-2-yl-Rest substituiert in 7-Stellung durch einen Halogenatom ist, in saurem Milieu behandelt und dann das erhaltene Produkt hydrolysiert.

12. Pharmazeutische Zusammensetzung, dadurch gekennzeichnet, daß sie mindestens ein Amid gemäß Anspruch 1 in reinem Zustand oder zusammen mit einem oder mehreren verträglichen und pharmazeutisch annehmbaren Verdünnungs- oder Hilfsmitteln enthält.

**Patentanspruch für die Vertragsstaaten: AT GR ES**

Verfahren zur Herstellung eines neuen substituierten Amids, dadurch gekennzeichnet, daß es der allgemeinen Formel:

R—CONH—Het (I)

entspricht, in welcher

das Symbol R entweder einen Phenylrest, substituiert durch einen Acyloxy-, Alkylthio- oder Alkyloxycarbonylaminorest, durch ein Chloratom in 2- oder 4-Stellung oder durch zwei Alkyloxyreste darstellt oder einen Heterocyclylrest, ausgewählt aus 2-Pyridyl, 4-Pyridyl, Pyrazinyl, 5,6-Dihydro-2-dithiinyl, 5,6-Dihydro-2-oxathiinyl, 1,3-Dithiolyl, Thiazolyl oder durch ein Halogenatom oder einen Alkylthiorest substituiertes Thienyl oder Furyl, darstellt oder R einen Alkenylrest mit 2 bis 4 Kohlenstoffatomen darstellt, dessen Doppelbindung sich in α-Stellung in bezug auf die Carbonylgruppe der Amidfunktion befindet, und das Symbol Het einen 1,8-Naphthyridin-2-ylrest, substituiert in 7-Stellung durch ein Halogenatom oder durch einen Alkyloxy-, Phenoxy-, 3-Chlorphenoxy-, Dichlorphenoxy-, Hydroxy- oder Cyanorest, darstellt,

oder das Symbol R einen Methoxyphenylrest darstellt und das Symbol Het einen 1,8-Naphthyridin-2-ylrest, substituiert in 7-Stellung durch einen 3-Chlorphenoxy-, Dichlorphenoxy-, Hydroxy- oder Cyanorest, darstellt,

oder das Symbol R einen Methoxy-3-pyridazinylrest darstellt und das Symbol Het einen 1,8-Naphthyridin-2-ylrest substituiert in 7-Stellung durch einen Phenoxyrest, darstellt,

wobei die oben genannten Alkyl- und Acylreste selbstverständlich 1 bis 4 Kohlenstoffatome in gerader oder verzweigter Kette enthalten, dadurch gekennzeichnet, daß man eine Säure der allgemeinen Formel:

R—COOH

worin R wie obige Bedeutung hat, oder ein reaktionsfähiges Derivat dieser Säure mit einem Amin der allgemeinen Formel:

Het —$NH_2$

worin Het die obige Bedeutung hat, umsetzt,

oder zur Herstellung eines substituierten Amids, worin das Symbol Het einen Hydroxysubstituenten enthält, dadurch gekennzeichnet, daß man das entsprechende substituierte Amid, worin Het ein 1,8-Naphthyridin-2-yl-rest, substituiert in 7-Stellung durch ein Halogenatom, ist, in saurem Milieu behandelt und dann das erhaltene Produkt hydrolysiert.

**Claims for the Contracting States: BE CH DE FR GB IT LI LU NL SE**

1. A new substituted amide which is of the general formula:

R—CONH—Het

in which:

either the symbol R denotes a phenyl radical substituted with an acyloxy, alkylthio or alkyloxycarbonylamino radical, with a chlorine atom in the 2- or 4-position or with two alkyloxy radicals, or denotes a heterocyclic radical chosen from 2-pyridyl, 4-pyridyl, pyrazinyl, 5,6-dihydrodithiin-2-yl, 5,6-dihydrooxathiin-2-yl, 1,3-dithiolyl or thiazolyl, or thienyl or furyl substituted with a halogen atom or with an alkylthio radical, or R denotes an alkenyl radical containing 2 to 4 carbon atoms in which the double bond is in the α-position with respect to the carbonyl group of the amide function, and the symbol Het denotes a 1,8-naphthyridin-2-yl radical substituted at the 7-position with a halogen atom or with an alkyloxy, phenoxy, 3-chlorophenoxy, dichlorophenoxy, hydroxy or cyano radical,

or the symbol R denotes a methoxyphenyl radical and the symbol Het denotes a 1,8-naphthyridin-2-yl radical substituted at the 7-position with a 3-chlorophenoxy, dichlorophenoxy, hydroxy or cyano radical,

or the symbol R denotes a methoxy-3-pyridazinyl radical and the symbol Het denotes a 1,8-naphthyridin-2-yl radical substituted at the 7-position with a phenoxy radical,

on the understanding that the alkyl and acyl radicals mentioned above contain 1 to 4 carbon atoms in a linear or branched chain.

2. A new amide according to claim 1, in which the symbol R denotes a phenyl radical substituted with an acyloxy, alkylthio or alkyloxycarbonylamino radical, with a chlorine atom in the 2- or 4-position or with 2 alkyloxy radicals, or denotes a hetercyclic radical chosen from 2-pyridyl, 4-pyridyl, pyrazinyl, 5,6-dihydrodithiin-2-yl, 5,6-dihydrooxathiin-2-yl, 1,3-dithiolyl, thiazolyl, 2-thienyl or 2-furyl substituted with a halogen atom at the 5-position, or 5-alkylthio-2-thienyl, or R denotes an alkenyl radical containing 2 to 4 carbon atoms in which the double bond is in the α-position relative to the carbonyl group of the amide function, and the symbol Het denotes a 1,8-naphthyridin-2-yl radical substituted at the 7-position with a halogen atom or with an alkyloxy or phenoxy radical,

or the symbol R denotes a methoxyphenyl radical and the symbol Het denotes a 1,8-naphthyridin-2-yl radical substituted at the 7-position with a 3-chlorophenoxy, 3,4-dichlorophenoxy, hydroxy or cyano radical,

or the symbol R denotes a 6-methoxy-3-pyridazinyl radical and the symbol Het denotes a 1,8-naphthyridin-2-yl radical substituted at the 7-position with a phenoxy radical,

on the understanding that the alkyl and acyl radicals mentioned above contain 1 to 3 carbon atoms in a linear or branched chain.

3. A new amide according to claim 1, in which

either the symbol R denotes a phenyl radical substituted with an acyloxy radical or denotes a heterocyclic radical chosen from 5,6-dihydrodithiin-2-yl, 1,3-dithiolyl or 4-thiazolyl, and the symbol Het denotes a 1,8-naphthyridin-2-yl radical substituted at the 7-position with a halogen atom or with a methoxy radical,

or the symbol R denotes a 4-methoxyphenyl radical and the symbol Het denotes a 1,8-naphthyridin-2-yl radical substituted at the 7-position with a 3-chlorophenoxy or cyano radical.

4. N-(7-Chloro-1,8-naphthyridin-2-yl)-4-acetoxybenzamide.

5. N-(7-Chloro-1,8-naphthyridin-2-yl)-3-acetoxybenzamide.

6. N-(7-Methoxy-1,8-naphthyridin-2-yl)-3-acetoxybenzamide.

7. N-(7-Chloro-1,8-naphthyridin-2-yl)-1,3-dithiol-4-carboxamide.

8. N-(Cyano-1,8-naphthyridin-2-yl)-4-methoxybenzamide.

9. A process for preparing a substituted amide according to claim 1, wherein the acid of general formula:

$$R—COOH$$

in which R is defined as in claim 1, or a reactive derivative of this acid, is reacted with an amine of general formula:

$$Het—NH_2$$

in which Het is defined as in claim 1.

10. A process according to claim 9, wherein a reactive derivative of the acid, chosen from the anhydride, a mixed anhydride, an acid halide or an ester, is reacted.

11. A process for preparing a substituted amide according to claim 1, for which the symbol Het contains a hydroxy substituent, wherein the corresponding substituted amide, defined according to claim 1, for which Het is a 1,8-naphthyridin-2-yl radical substituted at the 7-position with a halogen atom, is treated in acid medium, and the product obtained is then hydrolyzed.

12. A pharmaceutical composition, which contains at least one amide according to claim 1, in the pure state or in combination with one or more compatible and pharmaceutically acceptable diluents or adjuvants.

**Claim for the Contracting States: AT GR SP**

A process for the preparation of a new substituted amide which is of the general formula:

$$R—CONH—Het$$

in which:

either the symbol R denotes a phenyl radical substituted with an acyloxy, alkylthio or alkyloxycarbonylamino radical, with a chlorine atom in the 2- or 4-position or with two alkyloxy radicals, or denotes a heterocyclic radical chosen from 2-pyridyl, 4-pyridyl, pyrazinyl, 5,6-dihydrodithiin-2-yl, 5,6-dihydrooxathiin-2-yl, 1,3-dithiolyl or thiazolyl, or thienyl or furyl substituted with a halogen atom or with an alkylthio radical, or R denotes an alkenyl radical containing 2 to 4 carbon atoms in which the double bond is in the α-position with respect to the carbonyl group of the amide function, and the symbol Het denotes a 1,8-naphthyridin-2-yl radical substituted at the 7-position with a halogen atom or with an alkyloxy, phenoxy, 3-chlorophenoxy, dichlorophenoxy, hydroxy or cyano radical,

or the symbol R denotes a methoxyphenyl radical and the symbol Het denotes a 1,8-naphthyridin-2-yl radical substituted at the 7-position with a 3-chlorophenoxy, dichlorophenoxy, hydroxy or cyano radical,

or the symbol R denotes a methoxy-3-pyridazinyl radical and the symbol Het denotes a 1,8-naphthyridin-2-yl radical substituted at the 7-position with a phenoxy radical,

on the understanding that the alkyl and acyl radicals mentioned above contain 1 to 4 carbon atoms in a linear or branched chain, wherein an acid of the general formula:

$$R\text{—}COOH$$

in which R is defined as in claim 1, or a reactive derivative of this acid, is reacted with an amine of general formula:

$$Het\text{—}NH_2$$

in which Het is defined as above, or

for preparing a substituted amide for which the symbol Het contains a hydroxy substituent, wherein the corresponding substituted amide for which Het is a 1,8-naphthyridin-2-yl radical substituted at the 7-position with a halogen atom, is treated in acid medium, and the product obtained is then hydrolyzed.